# EUROPÄISCHE PATENTSCHRIFT

(11) **EP 2 079 391 B1**
(45) Veröffentlichungstag und Bekanntmachung des Hinweises auf die Patenterteilung: **27.02.2013**
(21) Anmeldenummer: 07818725.9
(22) Anmeldetag: 05.10.2007
(51) Int. Cl.: A61C 8/00, A61L 27/00, A61L 27/26, A61L 27/38

(54) **DREIDIMENSIONALE KÜNSTLICHE KALLUSDISTRAKTION**
THREE-DIMENSIONAL ARTIFICIAL CALLUS DISTRACTION
SÉPARATION DE CAL OSSEUX ARTIFICIELLE TRIDIMENSIONNELLE

(30) Priorität: 06.10.2006 DE 102006047248
(43) Veröffentlichungstag der Anmeldung: 22.07.2009
(73) Patentinhaber: CelGen AG, 6300 Zug (CH)
(72) Erfinder: HORVATH, Domonkos, 79798 Jestetten (DE)
(74) Vertreter: Schrell, Andreas
(86) Internationale Anmeldenummer: PCT/EP2007/008648
(87) Internationale Veröffentlichungsnummer: WO 2008/043484

(56) Entgegenhaltungen:
- EP-A- 1 523 956
- WO-A-00/69361
- WO-A-01/91663
- WO-A-03/048347
- WO-A-2006/088866
- US-A1- 2005 118 236
- US-B1- 6 280 191
- SARKAR ET AL: "Bone formation in a long bone defect model using a platelet-rich plasma-loaded collagen scaffold" BIOMATERIALS, ELSEVIER SCIENCE PUBLISHERS BV., BARKING, GB, Bd. 27, Nr. 9, März 2006 (2006-03), Seiten 1817-1823, XP005204036 ISSN: 0142-9612
- CANO ET AL: "Osteogenic alveolar distraction: A review of the literature" ORAL SURGERY, ORAL MEDICINE, ORAL PATHOLOGY, ORAL RADIOLOGY AND ENDODONTICS, MOSBY-YEAR BOOK, ST. LOUIS, MO, US, Bd. 101, Nr. 1, Januar 2006 (2006-01), Seiten 11-28, XP005208890 ISSN: 1079-2104

## Beschreibung

Die vorliegende Erfindung betrifft eine Vorrichtung zur Regeneration eines Knochens, insbesondere mittels dreidimensionaler Distraktion, Verfahren zur dreidimensionalen Kallusdistraktion und Verwendungen der genannten Vorrichtung.

Knochenverluste werden heutzutage in der Regel mit Knochenersatzmaterialien oder mit auto- oder allogenem Knochen ausgefüllt.

Bei den Knochenersatzmaterialien handelt es sich beispielsweise um anorganische Materialien wie Kalziumphosphate, Hydroxylapatit oder Biogläser (diese sollten nach langsamer Resorption mit Knochen ersetzt werden. Dies ist aber nur bei kleineren Defekten (sonst Infektionsgefahr wegen mangelnder Vaskularisierung) eher anwendbar. Die Resorption von anorganischen Materialien ist mangelhaft. Solche Knochenmaterialien, also Knochenersatzmaterialien, geben keine biomechanischen Impulse ab und lösen somit keine aktive Regeneration aus.), um synthetisch hergestellte organische Materialien, wie Polyester, Polyaminosäuren, Polyanhydride, Polyorthoester, Polyphosphazene, Polylactide oder Polyglycolide oder um allogene organische Materialien, die beispielsweise bovinen Ursprungs sind. Auch werden Materialkombinationen aus den verschiedenen Materialarten als Knochenersatzkomposite verwendet. Knochensubstanzverluste können aber auch mit mikravaskulären angeschlossenen autogenen oder allogenen vaskularisierten Transplantaten überbrückt werden. Die Verwendung eines allogenen Knochenersatzes kann jedoch unerwünschte Immunreaktionen auslösen und Infektionen übertragen.

Aus biologischer Sicht ist das beste Ersatzmaterial für einen Knochen ein autologes Spongiosatransplantat. Solche Transplantate sind jedoch nur limitiert verfügbar und zeigen eine hohe Resorptionsrate nach der Transplantation.

Die im Stand der Technik eingesetzten Materialien und Techniken liefern häufig eine ungenügende Knochenqualität, sodass beispielsweise Implantatlager nicht fest verankert sind. Darüber hinaus ist der Knochenersatz häufig nicht genügend vaskularisiert, wodurch die Infektionsgefahr erhöht ist. Auch werden bei Verfahren aus dem Stand der Technik häufig Wachstumsfaktoren verwendet, die die Kosten für die Verfahren stark erhöhen.

Anstelle der Verwendung eines Knochenersatzes kann fehlende Knochensubstanz teilweise auch durch Knochenregeneration aufgefüllt werden. Segmentale Unterbrechungen der knöchernern Kontinuität an langen Röhrenknochen können so mittels Distraktionsosteogenese behandelt werden.

Die Kallusdistraktion ist bereits seit über hundert Jahren bekannt. Der wichtigste biologische Reiz für die Knochenbildung ist die mechanische Beanspruchung. Dadurch werden piezoelektrische Kräfte freigesetzt, die Osteoblasten und Osteoklasten aktivieren. Die Distraktionsosteogenese induziert die Knochenneubildung, indem durch langsame Trennung von Knochensegmenten biologische Wachstumsreize ausgelöst werden. Durch diese Methode wird die direkte Bildung von Geflechtsknochen durch Distraktion erreicht. Die definierte Zugspannung bei der Knochengeneration ist wesentlich.

Legt man an Knochenfragmente eine solche definierte Zugspannung an, so zeigt das mesenchymale Gewebe im Spalt und an den angrenzenden Fragmentenden ein osteogenetisches Potential. Bei Vorliegen ausreichender vaskulärer Potenz kommt es unter progressiver Distraktion zur Metaplasie des organisierten Hämatoms, auch Blutkoagulat genannt, in einer Zone von longitudinal arrangiertem, fibrösem Gewebe, das sich unter optimalen externen und internen Bedingungen direkt in Geflechtsknochen umwandeln kann. Erschwerend ist jedoch, dass das Knochengewebe bei seiner Regeneration einer hoch-komplexen Steuerung unterliegt.

Die WO 01/91663 beschreibt eine zweidimensional orientierte Knochendistraktion mittels einer künstlichen Grenzfläche. Bei derartigen Distraktionsverfahren aus dem Stand der Technik ist häufig, beispielsweise im Kieferbereich, nur eine vertikale Regeneration möglich.

Auch die WO 03/048347 beschreibt eine Vorrichtung, die eine Zweidimensional orientierte Knochendistraktion bewirkt.

Somit ist eine Knochenregeneration durch Distraktion nicht bei jedem Knochendefekt anwendbar. Darüber hinaus sind die für eine Distraktion verwendeten Vorrichtungen komplex und Distraktionsverfahren dauern vergleichsweise lang.

Das der vorliegenden Erfindung zugrunde liegende technische Problem ist die Bereitstellung einer Vorrichtung, die es ermöglicht, Knochenregenerationsverfahren durchzuführen, die die Nachteile aus dem Stand der Technik überwinden. Das der Erfindung zugrunde liegende technische Problem ist auch die Bereitstellung von Vorrichtungen, Verwendungen derselben und Verfahren, die eine einfache und kostengünstige Knochenregeneration erlauben. Das der vorliegenden Erfindung zugrundeliegende technische Problem ist auch die Bereitstellung von Vorrichtungen und Verwendungen derselben.

Die vorliegenden Erfindung löst das ihr zugrunde liegende technische Problem insbesondere durch die Bereitstellung von Vorrichtungen, Verfahren und Verwendungen gemäß der Patentansprüche.

Die vorliegende Erfindung löst das ihr zugrunde liegende technische Problem insbesondere durch die Bereitstellung eines dreidimensionalen Gerüsts zur Regeneration eines Knochens, umfassend Gerüstfasern aus mindestens einem Gerüstmaterial und von diesen umschlossene Zwischenräume, wobei das Gerüstmaterial biokompatibel ist, vordefiniert und kontrolliert in Abhängigkeit von einer inneren oder äußeren Krafteinwirkung dehn- und/oder schrumpfbar ist, und wobei das Gerüst in Abhängigkeit der inneren oder äußeren Krafteinwirkung vordefiniert und kontrolliert sein Ausgangsvolumen ändern kann.

Durch die Bereitstellung des beschriebenen dreidimensionalen Gerüsts wird es ermöglicht, dieses in einen Knochendefekt einzubringen, beispielsweise chirurgisch einzubringen. Nach Einbringen in den Knochendefekt ist erfindungsgemäß vorgesehen, dass sich das Volumen des eingebrachten dreidimensionalen Gerüsts aufgrund seines Aufbaus und seiner Zusammensetzung entweder direkt und automatisch ohne weiteres Zutun oder nach Zuführen einer inneren oder äußeren Kraft ändert, zum Beispiel vergrößert oder verkleinert. Dies bewirkt, dass nach dem Einbringen des Gerüsts in den Knochendefekt eingewanderte und an den Gerüstfasern adhärierte osteogene Zellen oder Zellverbünde langsam und definiert, insbesondere sofern sie zu dem Gerüst einen distraktionswirksamen Abstand haben, einer Spannung, also einen biomechanischen Reiz, ausgesetzt werden, sodass in dem gesamten Defekt durch Distraktion auf einmal die Vorstufe eines Kallus entsteht, der lediglich noch verknöchern muss. Vorteilhafterweise wird dieser Reiz im Wesentlichen alle Zellen gleichzeitig erreichen. Erfindungsgemäß ist es möglich, auf die Osteoblasten direkt biomechanische Reize zu übertragen, ohne dass Fibroblasten notwendig sind. Die Distraktion kann also auf die Osteoblasten mit vergleichsweise kleinen Kräften wirken.

Das erfindungsgemäße Gerüst kann vorteilhafterweise in Verfahren, bevorzugt erfindungsgemäßen Verfahren, zur Knochenregeneration, insbesondere zur dreidimensionalen Kallusdistraktion verwendet werden.

Die vorliegende Lehre erfasst insbesondere Vorrichtungen und Verfahren zur Knochenregeneration, wobei vorzugsweise Knochen im Kieferbereich und/oder im Parodontalbereich, regeneriert werden sollen.

Insbesondere versteht die vorliegende Erfindung unter dem Begriff der Knochenregeneration auch die Regeneration von Knochendefekten zum Beispiel nach Zystektomie, Tumorchirurgie oder Unfallchirurgie etc., unabhängig von der Topografie, und/oder insbesondere auch die Regeneration kleinerer Knochendefekte, die zum Beispiel durch Parodontitis entstehen.

Im Zusammenhang mit der vorliegenden Erfindung wird unter einem "Gerüst" ein dreidimensionaler Körper verstanden. Das Gerüst umfasst sowohl aus einem Gerüstmaterial aufgebaute Gerüstfasern als auch Zwischenräume zwischen den Gerüstfasern. Die Gerüstfasern definieren die Form, Umrandung und Größe des Gerüsts und sind so aufgebaut und angeordnet, dass sie einen dreidimensionalen, ein bestimmtes Volumen aufspannenden Körper, das Gerüst, ergeben, das Zwischenräume, zum Beispiel Poren, Kavitäten, Spalten oder Hohlräume, zwischen den Gerüstfasern enthält. Das Volumen des Gerüsts ist, vorzugsweise ohne dass die strukturelle Identität des Gerüsts verändert wird, zum Beispiel ohne dass Gerüstmaterial entfernt oder hinzugefügt wird, durch Krafteinwirkung veränderbar, zum Beispiel plastisch oder elastisch veränderbar. Ein derartiges Gerüst kann zum Beispiel in Form eines Geflechts, porösen Kissens, schwammartigen Körpers oder einer Matrix vorliegen.

Im Zusammenhang mit der vorliegenden Erfindung wird unter "Zwischenräumen" eines erfindungsgemäßen dreidimensionalen Gerüsts das Volumen des Gerüsts verstanden, das nicht von einem festen Material, insbesondere von den Gerüstfasern, ausgefüllt wird und das innerhalb des Gesamtvolumens des Gerüsts liegt. Die Zwischenräume können unterschiedlichste Volumina aufweisen, die jedoch, auch in ihrer Summe, kleiner sind als das Gesamtvolumen des Gerüsts. Die Zwischenräume können jegliche Form aufweisen. Erfindungsgemäß bevorzugt sind die Zwischenräume Poren. Die Zwischenräume, zum Beispiel Poren, weisen vorzugsweise einen Durchmesser von 10 µm bis 10 mm, bevorzugt von 10 µm bis 1000 µm auf.

Erfindungsgemäß bevorzugt enthält das Gerüst Zwischenräume, zum Beispiel Poren mit einem Durchmesser von höchstens 100 µm. Erfindungsgemäß bevorzugt enthält das Gerüst Zwischenräume mit einem Durchmesser von über 450 µm. Erfindungsgemäß bevorzugt enthält das Gerüst sowohl Zwischenräume mit einem Durchmesser von unter 100 µm als auch Zwischenräume mit einem Durchmesser von über 450 µm. Das Gerüstmaterial, aus dem das Gerüst gebildet wird, kann Zellen als Trägerstruktur dienen. Dabei wird durch zum Beispiel Poren mit einer Porengröße von unterhalb 100 µm das Wachstum von Bindegewebe ermöglicht. Bei Poren mit einer Porengröße von über 450 µm können Blutgefäße in das Gerüst hineinwachsen und somit das Volumen, das das Gerüst einnimmt, vaskularisieren.

Erfindungsgemäß bevorzugt hat mindestens ein Teil der Zwischenräume, besonders bevorzugt der Poren, einen Durchmesser von 0,5 µm bis 5 µm. Erfindungsgemäß bevorzugt hat mindestens ein Teil der Zwischenräume, besonders bevorzugt der Poren, einen Durchmesser von 5 µm bis 25 µm. Erfindungsgemäß bevorzugt hat mindestens ein Teil der Zwischenräume, besonders bevorzugt der Poren, einen Durchmesser von 100 µm bis 1000 µm. Erfindungsgemäß bevorzugt hat ein Teil der Zwischenräume einen Durchmesser von 0,5 µm bis 5 µm, ein Teil der Zwischenräume einen Durchmesser von 5 µm bis 25 µm und ein Teil der Zwischenräume einen Durchmesser von 100 µm bis 1000 µm. Erfindungsgemäß bevorzugt hat ein Viertel, besonders bevorzugt ein Drittel, am meisten bevorzugt die Hälfte der Zwischenräume, besonders bevorzugt der Poren, einen Durchmesser von 0,5 µm bis 5 µm. Erfindungsgemäß bevorzugt hat ein Viertel, besonders bevorzugt ein Drittel, am meisten bevorzugt die Hälfte der Zwischenräume, besonders bevorzugt der Poren, einen Durchmesser von 5 µm bis 25 µm. Erfindungsgemäß bevorzugt hat ein Viertel, besonders bevorzugt ein Drittel, am meisten bevorzugt die Hälfte der Zwischenräume, besonders bevorzugt der Poren, einen Durchmesser von 100 µm bis 1000 µm.

Erfindungsgemäß bevorzugt beträgt der Durchmesser der Zwischenräume mindestens 450 µm und höchstens 5 mm, insbesondere bevorzugt mindestens 450 µm und höchstens 1000 µm.

Erfindungsgemäß bevorzugt hat das Gerüst offenporige Leitstrukturen, deren Porosität besonders bevorzugt 50% bis 70% beträgt, insbesondere 60% beträgt, und die das Einwachsen von Blutgefäßen ermöglichen.

Erfindungsgemäß bevorzugt haben die Zwischenräume, zum Beispiel Poren eine Größe, insbesondere einen Durchmesser, die/der es erlaubt, Dehnungsreize auf in den Poren befindliche Zellen von mindestens 0,5 µm, insbesondere von 1 µm, mehr bevorzugt von 2 µm, am meisten bevorzugt von 10 µm bis bevorzugt 100 µm, insbesondere besonders bevorzugt 1000 µm, besonders bevorzugt 1 cm, am meisten bevorzugt bis 10 cm zu übertragen. Erfindungsgemäß bevorzugt werden die Dehnungsreize mit einer Distraktionsfrequenz von höchstens 1 mm/Tag übertragen.

Im Zusammenhang mit der vorliegenden Erfindung wird unter dem "Volumen" eines erfindungsgemäßen dreidimensionalen Gerüsts das Volumen verstanden, das durch die die Außenflächen des Gerüsts definierenden Gerüstfasern begrenzt wird. Das Volumen des Gerüsts wird also durch das Volumen der Gerüstfasern und das Volumen der von den Gerüstfasern umschlossenen Zwischenräume gebildet. Das erfindungsgemäße dreidimensionale Gerüst liegt in bevorzugter Form in einem, vorzugsweise ursprünglichen, Ausgangsvolumen vor, das sich durch innere oder äußere Krafteinwirkung zu einem anderen Volumen ändern kann. Eine Änderung des Volumens bedeutet eine Änderung des Ausgangsvolumens, und zwar entweder eine Vergrößerung des Ausgangsvolumens, beispielsweise durch Dehnung des Gerüsts, oder eine Verkleinerung des Ausgangsvolumens, beispielsweise durch Kompression oder Schrumpfung des Gerüsts.

Erfindungsgemäß ist das Gerüstmaterial in Abhängigkeit von einer inneren, oder vorzugsweise äußeren, Krafteinwirkung vordefiniert und kontrolliert dehn- und/oder schrumpfbar. Das Material hat plastische oder elastische Eigenschaften. Diese Eigenschaften des Gerüstmaterials erlauben die erfindungsgemäß vorgesehene Kapazität des Gerüsts sein Volumen, reversibel oder irreversibel, vordefiniert und kontrolliert zu ändern.

Im Zusammenhang mit der vorliegenden Erfindung wird unter "vordefiniert und kontrolliert" eine Änderung des Ausgangsvolumens, insbesondere eine Dehnung oder eine Schrumpfung verstanden, die über eine vorbestimmte Strecke und/oder ein vorbestimmtes Volumen abläuft und deren Geschwindigkeit, also die Dehnungsgeschwindigkeit, Schrumpfungsgeschwindigkeit oder Volumenänderungsgeschwindigkeit ebenfalls vorbestimmt, das heisst bewusst gewählt ist. Erfindungsgemäß kann eine Änderung des Volumens auch lediglich eine Änderung der Form des Volumens sein. Erfindungsgemäß bevorzugt kann auch der Zeitpunkt des Dehnungs-, Schrumpfungs- oder Volumenänderungsbeginns vorbestimmt, das heisst bewusst ausgewählt werden.

Erfindungsgemäß bevorzugt kann das Ausgangsvolumen des dreidimensionalen Gerüsts durch eine von außen zugeführte Kraft verändert werden. Erfindungsgemäß bevorzugt wird die Kraft mittels eines Zugseils oder Zugstabs zugefügt. Erfindungsgemäß bevorzugt ist die zugeführte Kraft Wärme. Erfindungsgemäß bevorzugt ist die zugeführte Kraft Ultraschall. Erfindungsgemäß bevorzugt wird die Kraft durch einen Magneten zugeführt und ist daher eine elektromagnetische Kraft. Erfindungsgemäß bevorzugt kann die Kraft auch ein durch zum Beispiel von außerhalb des Gerüsts zugeführter Flüssigkeit oder Gas ausgeübter Druck sein.

Erfindungsgemäß bevorzugt kann das Gerüst sein Ausgangsvolumen dadurch ändern, dass eine Flüssigkeit, insbesondere interstitielle Flüssigkeit, aufgrund des colloidosmotischen Drucks in das Gerüstsystem hineindiffundiert. Ein notwendiges Konzentrationsgefälle wird erfindungsgemäß bevorzugt durch Dextrane und/oder Hydroxylethylstärke erreicht.

Im Zusammenhang mit der vorliegenden Erfindung wird unter einer "Dehnung" eine Vergrößerung des Gerüsts entlang mindestens einer Raumachse, bevorzugt entlang aller drei Raumachsen, verstanden. Eine Dehnung führt erfindungsgemäß bevorzugt zu einer Volumenvergrößerung des Gerüsts.

Im Zusammenhang mit der vorliegenden Erfindung wird unter einer "Schrumpfung" eine Verkleinerung des Gerüsts entlang mindestens einer Raumachse, bevorzugt entlang aller drei Raumachsen, verstanden. Erfindungsgemäß bevorzugt bewirkt die Schrumpfung eines Gerüsts eine Volumenverkleinerung des Gerüsts.

Erfindungsgemäß bevorzugt ist die Schrumpfung eine Kompression. Erfindungsgemäß bevorzugt ist also das Gerüstmaterial vordefiniert und kontrolliert in Abhängigkeit von einer inneren, oder vorzugsweise äußeren, Krafteinwirkung komprimierbar, so dass sich dergestalt auch das Volumen des Gerüsts ändern lässt. Im Zusammenhang mit der vorliegenden Erfindung wird unter einer "Kompression" eine Schrumpfung verstanden, bei der das Gerüst durch eine äußere Krafteinwirkung zusammengedrückt wird.

Erfindungsgemäß bevorzugt kann sich das Ausgangsvolumen durch Füllen der Zwischenräume des Gerüsts, besonders bevorzugt der Poren des Gerüsts, mit einer Flüssigkeit, besonders bevorzugt eine Flüssigkeit enthaltend Biomoleküle und/oder Zellen, am meisten bevorzugt Blut, vergrößern, bevorzugt durch einen durch die eingeflossene Flüssigkeit ausgeübten Druck von innen auf die Gerüstfasern.

Im Zusammenhang mit der vorliegenden Erfindung wird unter "Füllen der Zwischenräume" ein zumindest teilweises Einfließen einer Flüssigkeit in die Zwischenräume gemeint. Erfindungsgemäß bevorzugt werden die Zwischenräume durch das Füllen zum Großteil, am meisten bevorzugt ganz, mit der Flüssigkeit gefüllt.

Erfindungsgemäß bevorzugt weist das Gerüst zumindest ein, vorzugsweise bioabbaubares, Formelement auf. Erfindungsgemäß bevorzugt ist das Formelement eine Ummantelung. Erfindungsgemäß bevorzugt ist das Formelement eine Umschnürung, zum Beispiel ein Faden. Erfindungsgemäß bevorzugt ist das Formelement ein Kleber.

Erfindungsgemäß bevorzugt weist das Gerüst ein Formelement auf, aufgrund dessen sich das Gerüst in einem gedehnten Ausgangsvolumen befindet. Erfindungsgemäß bevorzugt weist das Gerüst mindestens ein Formelement auf, aufgrund dessen sich das Gerüst in einem komprimierten Ausgangsvolumen befindet. Erfindungsgemäß bevorzugt weist das Gerüst ein Formelement auf, aufgrund dessen sich das Gerüst in einem gedehnten oder komprimierten Ausgangsvolumen befindet und wobei sich das Ausgangsvolumen durch Entfernung des Formelements, vorzugsweise zwangsläufig und automatisch, ändern kann, insbesondere das Gerüst nicht mehr gedehnt oder komprimiert vorliegt.

Erfindungsgemäß bevorzugt ist das Material des Formelements ausgewählt aus der Gruppe bestehend aus Fibrin, Kollagen, mindestens einem Polysaccharid und Mischungen davon. Erfindungsgemäß bevorzugt ist das bioabbaubare Formelement Fibrin oder enthält dieses. Erfindungsgemäß bevorzugt ist das bioabbaubare Formelement Kollagen oder enthält dieses. Erfindungsgemäß bevorzugt ist das bioabbaubare Formelement mindestens ein Polysaccharid oder enthält dieses.

Erfindungsgemäß bevorzugt ist das Gerüst in seinem Ausgangsvolumen komprimiert oder geschrumpft. Erfindungsgemäß bevorzugt ist das Gerüst in seinem Ausgangsvolumen gedehnt oder expandiert. Erfindungsgemäß bevorzugt wird das Gerüst in zusammengestauchtem oder in auseinandergezogenem Zustand durch ein bioabbaubares Formelement in der jeweiligen Form gehalten.

Das Gerüst wird erfindungsgemäß bevorzugt nach der Herstellung zunächst mechanisch komprimiert oder expandiert. Erfindungsgemäß bevorzugt erfolgt die Kompression des Gerüsts durch Druck oder durch Trocknung und Schrumpfung. Im Anschluss daran wird es in bevorzugter Ausführung mit dem Formelement versehen, um so das komprimierte oder expandierte Ausgangsvolumen bis zum Beginn der erwünschten Distraktion beizubehalten.

Erfindungsgemäß verfügt das dreidimensionale Gerüst also über eine elastische Rückstellkraft. Dabei wird vor der Verwendung des Gerüsts das Ausgangsvolumen des Gerüst geändert, insbesondere komprimiert. In diesem Zustand erfolgt eine Fixierung mit einem bioabbaubaren Formelement, zum Beispiel einem Kleber. Wird dieses bioabbaubare Formelement, zum Beispiel der Kleber zersetzt, ändert sich das Volumen des Gerüsts durch die elastische Rückstellkraft, wodurch biomechanische Reize ausgelöst werden können.

Erfindungsgemäß bevorzugt enthält das dreidimensionale Gerüst eine aus bioabbaubarem Material hergestellte Feder. Die Feder wird erfindungsgemäß bevorzugt in komprimiertem oder gestrecktem Zustand mit einem bioabbaubaren Formelement, zum Beispiel einem Faden oder Kleber, fixiert. Wenn sich das Formelement durch Abbau, also Resorption, auflöst, bewirkt die in dem Gerüst integrierte Feder eine Formveränderung des Gerüsts, wobei dieses dabei biomechanische Reize abgibt.

Erfindungsgemäß bevorzugt ist die Resorptionszeit des Formelements kürzer, insbesondere erheblich kürzer, als die Resorptionszeit des Gerüsts.

Erfindungsgemäß bevorzugt beträgt die Resorptionszeit des Formelements mindestens einen Tag, besonders bevorzugt mindestens fünf Tage, am meisten bevorzugt mindestens sieben Tage. Erfindungsgemäß bevorzugt beträgt die Resorptionszeit des Formelements höchstens zwanzig Tage, mehr bevorzugt höchstens fünfzehn Tage, insbesondere zehn Tage, am meisten bevorzugt höchstens fünf Tage.

Erfindungsgemäß bevorzugt ist die Degradationskinetik des Gerüsts und des Gerüstmaterials an das Zeitschema einer mit dem erfindungsgemäßen Gerüst durchzuführenden Volumendistraktion angepasst.

Erfindungsgemäß bevorzugt ändert sich das Ausgangsvolumen des dreidimensionalen Gerüsts mit einer vorbestimmten Geschwindigkeit. Erfindungsgemäß bevorzugt ist die Geschwindigkeit, mit der sich das Ausgangsvolumen des Gerüsts ändern kann, höchstens so groß, dass die an dem Gerüst haftenden Zellen höchstens 1,5 mm/Tag, besonders bevorzugt 1,2 mm/Tag, insbesondere 1 mm/Tag, am meisten bevorzugt 0,9 mm/Tag distrahiert werden.

In einer bevorzugten Ausführungsform kann sich das Volumen vordefiniert und kontrolliert mit einer Geschwindigkeit ändern, bei der eine Dehnung oder Schrumpfung eines Volumens von 1000 µm³ bis 216000 µm³ in alle drei Raumkoordinaten von höchstens 0,6 mm pro Tag, besonders bevorzugt von höchstens 0,577 mm pro Tag, insbesondere von höchstens 0,55 mm pro Tag, am meisten bevorzugt von höchstens 0,5 mm pro Tag, erfolgt. In einer bevorzugten Ausführungsform kann sich das Volumen vordefiniert und kontrolliert mit einer Geschwindigkeit ändern, bei der eine Dehnung oder Schrumpfung eines Volumens von 1000 µm³ bis 216000 µm³ in alle drei Raumkoordinaten von mindestens 0,01 mm pro Tag, besonders bevorzugt von mindestens 0,1 mm pro Tag, insbesondere von mindestens 0,2 mm pro Tag, am meisten bevorzugt von mindestens 0,5 mm pro Tag, erfolgt.

In einer bevorzugten Ausführungsform kann sich das Volumen vordefiniert und kontrolliert mit einer Geschwindigkeit ändern, bei der eine Dehnung oder Schrumpfung eines zwischen 10 µm und 60 µm langen Abschnitts der Raumdiagonalen des Volumens des Gerüsts von höchstens 0,6 mm pro Tag, besonders bevorzugt von höchstens 0,577 mm pro Tag, insbesondere von höchstens 0,55 mm pro Tag, am meisten bevorzugt von höchstens 0,5 mm pro Tag, erfolgt. In einer bevorzugten Ausführungsform kann sich das Volumen vordefiniert und kontrolliert mit einer Geschwindigkeit ändern, bei der eine Dehnung oder Schrumpfung eines zwischen 10 µm und 60 µm langen Abschnitts der Raumdiagonalen des Volumens des Gerüsts von mindestens 0,01 mm pro Tag, besonders bevorzugt von mindestens 0,1 mm pro Tag, insbesondere von mindestens 0,2 mm pro Tag, am meisten bevorzugt von mindestens 0,5 mm pro Tag, erfolgt.

Erfindungsgemäß bevorzugt ist das Gerüst so gestaltet, dass die Änderung des Ausgangsvolumens kontinuierlich erfolgen kann. Erfindungsgemäß bevorzugt ist das Gerüst so gestaltet, dass die Änderungen des Ausgangsvolumens diskontinuierlich erfolgen kann.

Erfindungsgemäß bevorzugt ist das Gerüstmaterial, vorzugsweise sind die Gerüstfasern, nicht biogen, insbesondere enthalten kein Kollagen beziehungsweise sind kollagenfrei. Erfindungsgemäß bevorzugt ist das Gerüstmaterial biogen.

Erfindungsgemäß ist das Gerüstmaterial biokompatibel.

Erfindungsgemäß bevorzugt hat das Gerüstmaterial mindestens eine zelladhäsive Eigenschaft, das heisst ist in der Lage, Zellen, insbesondere Osteoblasten, Fibroblasten und/oder Endothelzellen, zu binden, vorzugsweise spezifisch und selektiv zu binden. Erfindungsgemäß bevorzugt wird die zelladhäsive Eigenschaft des Gerüstmaterials durch seine Oberflächenbeschaffenheit bestimmt.

Erfindungsgemäß bevorzugt ist das Gerüstmaterial und/oder das Gerüst bioabbaubar.

Im Zusammenhang mit der vorliegenden Erfindung bedeutet "bioabbaubar", dass das Material durch Hydrolyse, Polymerauflösung, enzymatische Degradation, und/oder Dissoziation der Materialbestandteile degradiert beziehungsweise resorbiert werden kann, vorzugsweise in einen Organsimus, zum Beispiel einem menschlichen oder tierischen Organismus. Erfindungsgemäß bevorzugt haben die Degradationsprodukte des Gerüstmaterials ein Molekulargewicht von höchstens 50000 g/mol, besonders bevorzugt von höchstens 40000 g/mol. Somit können sie auf normalem Weg ausgeschieden werden.

Erfindungsgemäß bevorzugt wird das bioabbaubare Gerüstmaterial in einem Organismus innerhalb einer Resorptionszeit von einem Jahr, besonders bevorzugt innerhalb von zwei Monaten, insbesondere innerhalb von einem Monat, am meisten bevorzugt innerhalb von zwei Wochen, abgebaut.

Erfindungsgemäß bevorzugt beginnt die Resorption nach 6 Wochen nach dem Einbringen des Gerüsts in einen Organismus.

Erfindungsgemäß besonders bevorzugt beträgt die Resorptionszeit des Gerüstmaterials und/oder des Gerüsts mindestens vier Wochen, besonders bevorzugt mindestens acht Wochen, insbesondere sechzehn Wochen, am meisten bevorzugt mindestens zweiunddreißig Wochen. Erfindungsgemäß bevorzugt beträgt die Resorptionszeit des Gerüstmaterials beziehungsweise des Gerüsts höchstens zweiundfünfzig Wochen, besonders bevorzugt höchstens achtunddreißig Wochen, stärker bevorzugt höchstens sechzehn Wochen, am meisten bevorzugt höchstens acht Wochen.

Erfindungsgemäß bevorzugt besteht das Gerüstmaterial aus mindestens einem Polymer oder enthält dieses, vorzugsweise aus räumlich vernetzten Polymeren. Durch den Zutritt von Wasser, Blut oder Serum bilden sich zum Beispiel Wasserstoffbrücken, Schwefelbrücken oder ähnliches, die die Polymerstruktur räumlich verändern, wodurch eine dreidimensionale Bewegung entsteht, die einen Impuls für eine biomechanische Reizübertragung auf die eingelagerten Zellen bewirkt.

Erfindungsgemäß bevorzugt ist das Gerüstmaterial gut verarbeitbar. Erfindungsgemäß bevorzugt ist das Gerüstmaterial und/oder das Gerüst sterilisierbar. Erfindungsgemäß bevorzugt lässt sich das Gerüst gut an die Regenerationsgeometrie anpassen. Erfindungsgemäß bevorzugt ist das Gerüstmaterial und/oder das Gerüst gut lagerfähig.

Erfindungsgemäß bevorzugt enthält das Gerüstmaterial ein Material, ausgewählt aus der Gruppe bestehend aus Polyglykolsäure, Polymilchsäure, Poly(ε-caprolacton), Poly(β-hydroxybutyrat), Poly(p-dioxanon), einem Polyanhydrid oder einer Mischung von diesen, zum Beispiel einer Mischung von Polymilchsäure und Polyglykolsäure.

Erfindungsgemäß bevorzugt enthält das Gerüstmaterial Copolymere, insbesondere aus zumindest zwei der vorgenannten Materialien. Erfindungsgemäß bevorzugt enthält das Gerüstmaterial Polymermischungen.

Erfindungsgemäß bevorzugt besteht das Gerüstmaterial aus einem Material, ausgewählt aus der Gruppe bestehend aus Polyglykolsäure, Polymilchsäure, Poly(ε-caprolacton), Poly(β-hydroxybutyrat), Poly(p-dioxanon), einem Polyanhydrid oder einer Mischung von diesen.

Erfindungsgemäß bevorzugt besteht das Gerüstmaterial aus Copolymeren aus zumindest zwei der vorgenannten Materialien.

Erfindungsgemäß bevorzugt besteht das Gerüstmaterial aus Polymilchsäure und Polyglykolsäure. Erfindungsgemäß bevorzugt liegen die Polymilchsäure und Polyglykolsäure als Copolymer vor.

Erfindungsgemäß bevorzugt ist das Gerüstmaterial Polyglykolsäure oder enthält diese. Erfindungsgemäß bevorzugt ist das Gerüstmaterial Polymilchsäure oder enthält diese. Erfindungsgemäß bevorzugt ist das Gerüstmaterial Poly(ε-caprolacton) oder enthält dieses. Erfindungsgemäß bevorzugt ist das Gerüstmaterial Poly(β-hydroxybutyrat) oder enthält dieses. Erfindungsgemäß bevorzugt ist das Gerüstmaterial Poly(p-dioxanon) oder enthält dieses. Erfindungsgemäß bevorzugt ist das Gerüstmaterial mindestens ein Polyanhydrid oder enthält dieses. Erfindungsgemäß bevorzugt können auch andere geeignete Materialien verwendet werden.

Erfindungsgemäß bevorzugt besteht das Gerüstmaterial aus mindestens einem Polylactit und mindestens einem Polyglykolid.

Erfindungsgemäß bevorzugt können durch die Kombination und Variation von Lactit- und Glykolidanteilen Copolymere mit unterschiedlichen physikalischen und mechanischen Eigenschaften hergestellt und als Gerüstmaterial eingesetzt werden.

Erfindungsgemäß bevorzugt hat das Gerüstmaterial bestimmte gummielastische Eigenschaften und ist mechanisch stabil genug, um den im Defektbereich des Knochens herrschenden Gewebsdruck zu überwinden. Insbesondere ist das Gerüstmaterial und/oder das Gerüst in bevorzugter Ausführungsform in der Lage, einen effektiven Gewebsdruck des ihn umgebenden Gewebes von bis zu 9.5 mm Hg zu widerstehen.

Erfindungsgemäß bevorzugt ist das Gerüstmaterial anisotrop. Im Zusammenhang mit der vorliegenden Erfindung wird unter "Anisotropie" die räumliche Variation der makroskopischen mechanischen Eigenschaften verstanden.

Erfindungsgemäß bevorzugt ist die Oberfläche des Gerüstmaterials, insbesondere durch Anordnung von Konturen, vergrößert. Diese Vergrößerung erhöht nicht nur die für die Zellen verfügbare Oberfläche, sondern beeinflusst auch die Organisation des Zellwachstums.

Erfindungsgemäß bevorzugt bestimmen die Größe und räumliche Verteilung der Zwischenräume, insbesondere der Poren, in dem Gerüst deren Zugänglichkeit für Zellen sowie den Austausch von Nährstoffen. Mit zunehmender metabolischer Aktivität der Zellen nimmt auch die Dichte der Blutgefäße zu, um die Stoffaustauschstrecken durch Diffusion und Osmose zwischen den aktiven Zellen und dem Blut minimal zu halten. Erfindungsgemäß bevorzugt ist es, den Stoffaustausch durch die Bereitstellung geeigneter vaskularisierender Makroporosität zu erreichen. In einem feinporigen Gerüst muss der Stoffaustausch große Distanzen überwinden, damit wird die Diffusion zum limitierenden Faktor.

Erfindungsgemäß weisen die Gerüstfasern eine Dicke von 50 µm bis 3000 µm, bevorzugt von 60 µm bis 2000 µm, insbesondere von 100 µm bis 1000 µm, auf. Erfindungsgemäß bevorzugt hat das Gerüstmaterial, insbesondere die Gerüstfasern, einen Durchmesser von 5 - 50 µm.

Erfindungsgemäß bevorzugt hat das Gerüstmaterial, insbesondere die Gerüstfasern, eine Dichte von 1 - 5 g/cm³. Erfindungsgemäß bevorzugt hat das Gerüstmaterial, insbesondere die Gerüstfasern, eine Steifigkeit, Dehnbarkeit, Festigkeit von 1000 - 8000 MPa. Erfindungsgemäß bevorzugt hat das Gerüstmaterial, insbesondere die Gerüstfasern, ein E-Modul von 50 - 500 GPa. Erfindungsgemäß bevorzugt hat das Gerüstmaterial, insbesondere die Gerüstfasern, eine Bruchdehnung von 0,2 - 10%.

Efindungsgemäß bevorzugt enthält das Gerüst vor dem Einbringen in einen Defektbereich eines Knochens in den Zwischenräumen Zellen, insbesondere Endothelzellen und/oder Osteoblasten und/oder Fibroblasten.

Erfindungsgemäß bevorzugt besteht das Gerüstmaterial aus mindestens einem Faserverbundwerkstoff oder enthält diese. Erfindungsgemäß bevorzugt besteht das Gerüstmaterial aus Fasern eines Faserverbundwerkstoffs oder enthält diese. Erfindungsgemäß bevorzugt ist das Gerüstmaterial optional von einer thermoplastischen Matrix ummantelt oder in diese eingebettet. Erfindungsgemäß wird dadurch ein mechanischer Schutz der Fasern bei Druck- und Scherbeanspruchung, Festigkeit bei Beanspruchung und Schutz des Empfängergewebes vor den integrierten Faserpartikeln bereitgestellt. Die Erfindung sieht insbesondere optional eine Versiegelung der 3D-Fasergerüstoberfläche vor. Die Fasern können bevorzugt in eine Matrix mit zum Beispiel unterschiedlicher Schichtdicke eingebettet sein. Erfindungsgemäß bevorzugt sind in dem Gerüstmaterial Fasern eines Faserverbundswerkstoffs in einer Polymermatrix ganz oder teilweise eingebettet.

Erfindungsgemäß bevorzugt ist das Gerüstmaterial beschichtet. Erfindungsgemäß bevorzugt ist das Gerüstmaterial mittels Dünnschichttechnologie beschichtet. Erfindungsgemäß bevorzugt ist das Gerüstmaterial mittels Vakuum-, Plasma- oder Ionentechnik beschichtet. Durch eine erfindungsgemäß bevorzugte Beschichtung kann eine erwünschte Proteinadsorption gezielt beeinflusst werden. Weiterhin kann durch die Beschichtung mit antithrombogenen Oberflächen eine Verbesserung der Blutverträglichkeit erzielt werden. Durch eine erfindungsgemäß bevorzugte dünne Beschichtung kann eine Zelladhäsion an das Gerüstmaterial und eine Beeinflussung des Zellwachstums der adhärierten Zellen gezielt gesteuert werden. Durch eine erfindungsgemäß bevorzugte Beschichtung können die elektrischen Eigenschaften der Oberfläche des Gerüstmaterials gezielt verändert werden.

Erfindungsgemäß bevorzugt können die Fasern eines Faserverbundwerkstoffs beschichtet werden, damit die Zelladhäsion erhöht wird. Erfindungsgemäß bevorzugt sind die Fasern mit Titan beschichtet. Erfindungsgemäß bevorzugt ist das Gerüstmaterial mit Titan beschichtet. Erfindungsgemäß bevorzugt sind die Fasern mit Titanoxid beschichtet. Erfindungsgemäß bevorzugt ist das Gerüstmaterial mit Titanoxid beschichtet. Erfindungsgemäß bevorzugt sind die Fasern mit Natriumalginat beschichtet. Erfindungsgemäß bevorzugt ist das Gerüstmaterial mit Natriumalginat beschichtet.

Erfindungsgemäß bevorzugt sind die Fasern mit einem Hydrogel beschichtet. Erfindungsgemäß bevorzugt ist das Gerüstmaterial mit einem Hydrogel beschichtet. Die Hydrogelbeschichtung kann erfinsungsgemäß bevorzugt dazu genutzt werde, dass sich das Volumen des Gerüsts nicht sofort nach Einbringen des Gerüst in einen Knochendefekt ändert, sondern erst zu einem späteren Zeitpunkt, besonders bevorzugt nach einer Woche.

Um eine möglichst effektive Verwendung des erfindungsgemäßen Gerüsts zu ermöglichen, müssen Osteoblasten gut an die Gerüstfasern binden können. Durch eine verbesserte Adhäsion zwischen Gerüst und Osteoblasten werden bei der Verwendung eines erfindungsgemäßen Gerüsts, insbesondere in einem erfindungsgemäßen Verfahren, mehr Osteoblasten durch das Gerüst mit einem oder mehreren biomechanischen Impulsen aktiviert Daher ist erfindungsgemäß bevorzugt vorgesehen das Gerüstmaterial oder die Beschichtung des Gerüstmaterials so zu gestalten, dass eine möglichst gute Osteoblastenbindung an das Gerüst erfolgen kann. Die Adhäsionsbindung der Osteoblasten an das Gerüst ist erfindungsgemäß bevorzugt so stark, dass die Bindung während eines Teils der Volumenausdehnung, besonders bevorzugt während der gesamten Volumenausdehnung, des Gerüst, insbesondere bei Verwendung des Gerüsts in einem erfindungsgemäßen Verfahren, aufrechterhalten wird.

Erfindungsgemäß bevorzugt sind die Fasern glatt. Erfindungsgemäß bevorzugt ist das Gerüstmaterial glatt. Erfindungsgemäß bevorzugt ist die Beschichtung des Gerüstmaterials glatt. Erfindungsgemäß bevorzugt sind die Fasern rau. Erfindungsgemäß bevorzugt ist das Gerüstmaterial rau. Erfindungsgemäß bevorzugt ist die Beschichtung des Gerüstmaterials rau. Durch eine erfindungsgemäß bevorzugte raue Oberfläche steht eine größere Oberfläche zur Bindung der Osteoblasten zur Verfügung.

Erfindungsgemäß bevorzugt sind die Fasern mit Hydroxylapatit beschichtet. Erfindungsgemäß bevorzugt ist das Gerüstmaterial Hydroxylapatit beschichtet. Eine erfindungsgemäß bevorzugte Beschichtung mit Hydroxylapatit ermöglicht eine bindungsfördernde Adsorption von Proteinen.

Erfindungsgemäß bevorzugt sind die Fasern mit einem Hydrogel beschichtet. Erfindungsgemäß bevorzugt ist das Gerüstmaterial mit einem Hydrogel beschichtet. Erfindungsgemäß bevorzugt ist die die Hydrogelschicht dünn.

Erfindungsgemäß bevorzugt sind die Fasern mit mindestens einem Protein beschichtet. Erfindungsgemäß bevorzugt ist das Gerüstmaterial mit mindestens einem Protein beschichtet. Erfindungsgemäß bevorzugt enthält das mindestens eine Protein die Aminosäuresequenz Arg-Gly-Asp, also RGD. Erfindungsgemäß bevorzugt sind die Fasern mit mindestens einem Peptid beschichtet. Erfindungsgemäß bevorzugt ist das Gerüstmaterial mit mindestens einem Peptid beschichtet. Erfindungsgemäß bevorzugt ist das mindestens eine Peptid ein Peptid, das die Zelladhäsion initiiert. Erfindungsgemäß bevorzugt ist das mindesten eine Peptid ein RGD-Peptid. Erfindungsgemäß bevorzugt ist das mindestens eine Peptid synthetisch hergestellt. Erfindungsgemäß bevorzugt enthält das mindestens eine Peptid die Aminosäuresequenz Arg-Gly-Asp, also RGD. Erfindungsgemäß bevorzugt Besteht das mindestens eine Peptid die Aminosäuresequenz Arg-Gly-Asp, also RGD.

Erfindungsgemäß bevorzugt sind die Fasern mit sternförmigen Polyethylen-Glykol-Polymeren (Star-PEG) beschichtet. Erfindungsgemäß bevorzugt ist das Gerüstmaterial mit sternförmigen Polyethylen-Glykol-Polymeren (Star-PEG) beschichtet.

Erfindungsgemäß bevorzugt ist das mindestens eine Protein an die Polyethylen-Glykol-Polymerbeschichtung, besonders bevorzugt kovalent, gebunden. Erfindungsgemäß bevorzugt ist das mindestens eine Peptid an die Polyethylen-Glykol-Polymerbeschichtung besonders bevorzugt kovalent, gebunden.

Die Adhäsion von Osteoblasten ist ein Rezeptor-vermittelter Kontakt zwischen den Molekülen der Extrazellulären Matrix und den Aktinfasern des Cytoskeletts. Diese Region wird auch als fokale Kontaktzone bezeichnet. In den fokalen Kontakten sind sowohl Moleküle, die für eine Bindung sorgen, als auch Moleküle, die für eine Slgnaltransduktion verantwortlich sind. Die Ausbildung der fokalen Adhäsion ist hauptsächlich durch Integrine bedingt. Die Integrine unterscheiden sich durch ihre Bioaffinität von anderen Zelloberflächenrezeptoren. Adhäsionsproteine in Form von ultradünner Beschichtung an dem Gerüstmaterial erleichtern die Adhäsionsbindung von Osteoblasten an das erfindungsgemäße Gerüst. Fibronektin ist ein extrazelluläres Adhäsionsprotein mit mehreren spezifischen Bindungsstellen für Rezeptoren und dient somit zur Bindung der Osteoblasten an die extrazelluläre Matrix. Fibronektin ist ein großes Glykoprotein, dass als Dimer aus zwei nahezu identischen Untereinheiten besteht. Fibronektin besteht aus etwa 90 Aminosäuren. Die Zellbindende Stelle von Fibronektin wurde als Tripeptidsequenz Arg-Gly-Asp (RGD) identifiziert.

Die Eigenschaften der Faserverbundwerkstoffe mit oder ohne Matrix können erfindungsgemäß bevorzugt durch den Faservolumengehalt und durch die Orientierung der Fasern in der Faserarchitektur bestimmt werden. Dadurch kann auch erfindungsgemäß bevorzugt die Festigkeit und das E-Modul des erfindungsgemäßen Gerüstes bestimmt werden.

Besteht das Gerüst erfindungsgemäß bevorzugt aus Fasern eines Faserverbundwerkstoffs oder enthält das Gerüst solche Fasern, so ist das Gerüst erfindungsgemäß bevorzugt so aufgebaut, dass eine Beanspruchung in Faserrichtung oder senkrecht zur Faserrichtung erfolgen kann. Erfindungsgemäß bevorzugt kann das Gerüst aber auch derart aufgebaut sein, dass Beanspruchungen in verschiedenen Richtungen in Bezug auf die Faserrichtung erfolgen können. Erfindungsgemäß bevorzugt werden die Fasern bei einer Beanspruchung senkrecht zur Faserrichtung in Serie angeordnet. Wird erfindungsgemäß bevorzugt eine Matrix zwischen den Fasern verwendet, so wird diese dabei stärker beansprucht.

Erfindungsgemäß bevorzugt besteht das dreidimensionale Gerüst aus einem Faserverbund aus Endlosfasern oder enthält diesen.

Erfindungsgemäß bevorzugt ist ein Gerüst aus einem Faserverbundwerkstoff aus unterschiedlich orientierten Schichten aufgebaut. Erfindungsgemäß bevorzugt kann die Schichtabfolge der Fasern symmetrisch zur Mittelebene des Gerüsts oder zufällig oder in Zwischenstufen davon erfolgen.

Erfindungsgemäß bevorzugt stellen Fasern eines Faserverbundwerkstoffs das hauptlasttragende Element eines Gerüsts mit thermoplastischer Matrix dar. Aufgrund ihres höheren E-Moduls und höherer Festigkeit bestimmen die Fasern weitergehend die mechanischen Eigenschaften des Verbundes.

Erfindungsgemäß bevorzugt enthält das Gerüst eine Faserarchitektur in Form eines Vlieses, eines 2D-Gerüsts, eines multiaxialen Gerüsts und/oder eines 3D-Geflechts.

Erfindungsgemäß bevorzugt wird als Faserarchitektur für das Gerüst ein undirektionales Gewebe verwendet.

Erfindungsgemäß bevorzugt ist die Faserarchitektur des Gerüsts ein biaxiales Gewebe. Erfindungsgemäß bevorzugt ist die Faserarchitektur des Gerüsts ein Gestrick. Erfindungsgemäß bevorzugt ist die Faserarchitektur des Gerüsts ein multiaxiales, mehrlagig gewirktes Gewebe.

Erfindungsgemäß bevorzugt zeichnet sich das Gerüst durch seine Biofunktionalität aus. Bei der Biofunktionalität sind die physikalischen, mechanischen und/oder biologischen Eigenschaften in Funktion mit der zeitlichen biomechanischen Reizabgabe wichtig.

Die Auswahl bestimmter erfindungsgemäß bevorzugter Architekturen, insbesondere Gestricktechniken, für den Aufbau des Gerüsts aus dem Gerüstmaterial erlaubt es, die Dichte, Verteilung und Orientierung des Gerüstmaterials zu kontrollieren und ist ein effektives Mittel um Strukturen zu gestalten, die das mechanische Verhalten und das Einwachsen von Zellen in das Gerüst steuerbar machen.

Erfindungsgemäß bevorzugt ist die Oberfläche des Gerüstmaterials chemisch modifiziert. Erfindungsgemäß bevorzugt ist die Oberfläche des Gerüstmaterials durch reaktive Moleküle oder Molekülgruppen chemisch modifiziert. Erfindungsgemäß bevorzugt können die Moleküle oder Molekülgruppen, mit denen die Oberfläche des Gerüstmaterials chemisch modifiziert ist, mit Ankerproteinen der extrazellulären Matrix von Zellen reagieren. Erfindungsgemäß bevorzugt ist die Oberfläche des Gerüstmaterials hydrophil. Hydrophile Oberflächen erlauben eine bessere Adhäsion für Zellen als hydrophobe. Die Oberflächen jedes Gerüsts korrespondiert mit seiner Polarisierbarkeit.

Die Erfindung betrifft auch ein dreidimensionales Gerüst, das für eine bestimmte Zeitdauer in der Lage ist, zu pulsieren oder zu vibrieren, zum Beispiel angeregt durch ein Magnetfeld oder Ultraschall, und dadurch biomechanische Reizimpulse für osteogene Zellen abgeben kann.

Erfindungsgemäß bevorzugt können die Strukturelemente des Gerüsts nach stochastischem, fraktalem oder periodischem Prinzip angeordnet sein.

Erfindungsgemäß bevorzugt ist das Gerüst aus Substrukturen und aus einfachen Subsystemen aufgebaut. Erfindungsgemäß bevorzugt kann die Komplexität des Gerüsts durch hierarchische Abfolge von Strukturelementen über mehrere Stufen erhöht liegen, in dem die kleinste funktionierende Einheit zu Gruppen und diese wiederum mit weiteren Gruppen anderer Funktionalität zu größeren Einheiten zusammengefasst werden.

Erfindungsgemäß bevorzugt kann eine Mehrzahl von Gerüsten auch in Form eines Granulats vorliegen. Dabei sind die Grundeinheiten des Granulats, also die einzelnen Gerüste, identisch oder ähnlich aufgebaut, aber nicht in einer Superstruktur zusammengefasst. Erfindungsgemäß bevorzugt sind die einzelnen Granulatpartikel, also die einzelnen Gerüste, mit einem bioabbaubaren Kleber miteinander fixiert und können so in den Defekt eingebaut werden.

Die Erfindung betrifft auch die Verwendung mindestens eines im Rahmen der vorliegenden Lehre beschriebenen erfindungsgemäßen dreidimensionalen Gerüsts, gegebenenfalls in einer bevorzugten Ausführungsform enthaltend eine Feder, zur Herstellung eines Kits zur Knochenregeneration. Die Erfindung betrifft auch die Verwendung eines erfindungsgemäßen aus Gerüsten bestehenden Granulats zur Herstellung eines Kits zur Knochenregeneration. Erfindungsgemäß bevorzugt enthalten die genannten Kits mindestens ein chirurgisches Instrument, besonders bevorzugt mindestens einen Applikator, zum Beispiel eine Spritze, und eine Kapsel zur Aufnahme des Gerüsts, zum Beispiel des Gerüsts in Granulatform. Erfindungsgemäß bevorzugt enthält der Kit eine Gebrauchsanleitung. Erfindungsgemäß bevorzugt enthält der Kit eine Verpackung, besonders bevorzugt eine Verpackung, die eine sterile Lagerung des Gerüsts ermöglicht. Erfindungsgemäß bevorzugt enthält der Kit einen Kleber, insbesondere einen Kleber zum Fixieren des Gerüsts in einem Knochendefekt.

In einer weiteren Ausführungsform der Erfindung betrifft diese die Verwendung eines erfindungsgemäßen dreidimensionalen Gerüsts zur Herstellung eines Kits zur Knochenregeneration, wobei der Kit zusätzlich ein Formelement der vorgenannten Art, zum Beispiel eine Ummantelung, einen Kleber oder eine Umschnürung, aufweist.

Die Erfindung betrifft auch die Verwendung eines biokompatiblen Gerüstmaterials, das vordefiniert und kontrolliert in Abhängigkeit von einer inneren oder äußeren Krafteinwirkung dehn- und/oder schrumpfbar ist und eine zelladhäsive Eigenschaft hat, zur Herstellung eines dreidimensionalen Gerüsts, umfassend ein Gerüstmaterial und von diesem umschlossene Zwischenräume, zur Regeneration eines Knochens, wobei das dreidimensionale Gerüst in einen Defektbereich eines Knochens eingebracht werden kann.

Die Erfindung betrifft auch die Bereitstellung eines Verfahrens zur Herstellung eines dreidimensionalen Gerüsts zur Regeneration eines Knochens, wobei aus einem biokompatiblen Gerüstmaterial, das vordefiniert und kontrolliert in Abhängigkeit von einer inneren oder äußeren Krafteinwirkung dehn- und/oder schrumpfbar ist und eine zelladhäsive Eigenschaft hat, ein Gerüst geformt wird, das in Abhängigkeit der inneren oder äußeren Krafteinwirkung vordefiniert und kontrolliert sein Ausgangsvolumen ändern kann.

Die Erfindung betrifft insbesondere ein Verfahren zur Herstellung eines erfindungsgemäßen dreidimensionalen Gerüsts, wobei Gerüstfasern aus mindestens einem nicht-biogenen Gerüstmaterial in einem Fließpressverfahren zu einem Gerüst geformt werden, wobei das Gerüstmaterial biokompatibel ist, vordefiniert und kontrolliert in Abhängigkeit von einer Krafteinwirkung dehn- und/oder schrumpfbar ist und eine zelladhäsive Eigenschaft hat.

Erfindungsgemäß bevorzugt können die Zwischenräume, die von dem Gerüstmaterial umschlossen werden, durch Herauslösen von Salzen aus Salz-Polymermischungen hergestellt werden.

Erfindungsgemäß bevorzugt können zur Herstellung der Gerüstfasern Filze aus Polymerfasern durch Verkleben der Fasern im Gerüstmaterial stabilisiert werden. Erfindungsgemäß bevorzugt kann das Gerüstmaterial, insbesondere ein Polymer, durch thermisch aktivierte Schäummittel oder durch Druckexpansion geschäumt werden. Erfindungsgemäß bevorzugt werden die Zwischenräume, die von dem Gerüstmaterial umschlossen werden, durch einen Sol-Gel-Prozess hergestellt.

Die Erfindung löst das ihr zugrunde liegende technische Problem auch und betrifft daher auch die Bereitstellung eines Verfahrens zur Regeneration eines Knochens, wobei mindestens ein vorgenanntes dreidimensionales Gerüst, insbesondere umfassend ein Gerüstfasern ausbildendes Gerüstmaterial und von den Gerüstfasern umschlossene Zwischenräume, in einen Defektbereich eines Knochens eingebracht wird, wobei das Gerüstmaterial biokompatibel ist, vordefiniert und kontrolliert in Abhängigkeit von einer Krafteinwirkung dehn- und/oder schrumpfbar ist und eine zelladhäsive Eigenschaft, insbesondere für Osteoblasten, Fibroblasten und/oder Endothelzellen hat, und wobei das Gerüst einer inneren oder äußeren Krafteinwirkung ausgesetzt wird und sich das Ausgangsvolumen des Gerüsts in Abhängigkeit der Krafteinwirkung vordefiniert und kontrolliert ändert.

Im Rahmen des erfindungsgemäßen Verfahrens zur Knochenregeneration wird demgemäss in einer bevorzugten Ausführungsform ein dreidimensionales Gerüst in einen Defektbereich eines Knochens eingebracht. In diesem Defektbereich wird das Gerüst von einem Blutkoagel eingeschlossen, das heißt die Oberflächen des Gerüsts kontaktieren sowohl im Gerüstinneren wie auch an den Außenflächen die im Blutkoagel enthaltenen autologen Zellen. Die vom Gerüstmaterial umschlossenen Zwischenräume werden, erfindungsgemäß bevorzugt zumindest teilweise, besonders bevorzugt größtenteils, insbesondere ganz durch das Blutkoagel ausgefüllt. Da das Gerüst dreidimensional aufgebaut ist, kann die Gerüstoberfläche im gesamten Raum, der durch das Gerüst ausgefüllt wird, mit dem im Blutkoagel enthaltenen autologen Zellen in Kontakt kommen. Nach dem Einbringen des Gerüsts in den Defektbereich eines Knochens wird eine Volumenveränderung, also insbesondere eine Volumenverkleinerung oder -vergrößerung, des Gerüsts ausgelöst, zum Beispiel durch Einwirkung einer äußeren oder inneren Kraft, zum Beispiel auch durch Entfernung eines Formelements. Diese führt zu der erwünschten biomechanischen Reizung der eingelagerten osteogenen Zellen und so zur Distraktion und damit Knochenregeneration. Erfindungsgemäß bevorzugt erfolgt die Krafteinwirkung innerhalb des Körpers, insbesondere innerhalb des Knochendefekts. Erfindungsgemäß bevorzugt erfolgt die Krafteinwirkung auf ein Formelement, zum Beispiel einen Kleber oder einen Faden, der das dreidimensionale Gerüst in einem komprimierten und/oder gedehnten Zustand fixiert. Erfindungsgemäß bevorzugt wird das Formelement, zum Beispiel der Kleber oder der Faden, durch eine äußere Krafteinwirkung zerstört, insbesondere biologisch abgebaut. Durch die Zerstörung des Formelements, zum Beispiel des Klebers oder Fadens kann das dreidimensionale Gerüst in den ungedehnten und/oder nicht komprimierten Zustand zurückkehren, wodurch sich das Volumen des dreidimensionalen Gerüsts verändert.

Erfindungsgemäß bevorzugt wird das Volumen des Gerüsts in Abhängigkeit einer äußeren Krafteinwirkung verändert. Erfindungsgemäß bevorzugt erfolgt die äußere Krafteinwirkung außerhalb des Körpers. Erfindungsgemäß bevorzugt erfolgt die äußere Krafteinwirkung durch Seilzüge und/oder Stäbe. Erfindungsgemäß bevorzugt erfolgt die Krafteinwirkung durch mindestens einen Magneten. Erfindungsgemäß bevorzugt erfolgt die Krafteinwirkung durch Ultraschall.

Erfindungsgemäß bevorzugt kann die Volumenänderung des Gerüsts in verschiedenen Größenordnungen liegen. Sie liegt bevorzugt bei etwa 10 % der Längenausdehnung der eingeschlossenen Zellen beziehungsweise Zellgruppen.

Bei einer Porengröße von 100 µm werden einzelne Zellen, zum Beispiel Osteoblasten, in dem Gerüst eingeschlossen. Bei einer Zellgröße von 10 µm beträgt die tägliche räumliche Dehnungsstrecke bevorzugt mindestens 1 µm (Untergrenze), und zwar etwa 10 % der Zellgröße.

Bei einer erfindungsgemäßen Porengröße von 100 - 1000 µm werden vorzugsweise gemischte Zellkolonien in den Poren zum Beispiel Osteoblasten, Fibroblasten, etc. eingeschlossen. Bevorzugt ist dann eine tägliche Dehnungsstrecke von 10 bis 100 µm, ebenfalls etwa 10 %, hier der Koloniegröße.

Bei einer erfindungsgemäßen Porengröße von 1000 µm - 10 mm werden vorzugsweise zusammenhängende Gewebszellen von Bindegewebe, Kallus, Vorstufe von Kallus, o.ä. eingeschlossen. Die tägliche räumliche Dehnungszunahme beträgt bevorzugt 0,3 - 1 mm (Obergrenze), ebenfalls etwa 10 %, hier der Gewebegröße.

Erfindungsgemäß bevorzugt beträgt die Änderung der Dehnungsstrecke mindestens 0,5 µm, besonders bevorzugt mindestens 1 µm, mehr bevorzugt mindestens 10 µm, noch mehr bevorzugt mindestens 100 µm, sehr bevorzugt mindestens 1000 µm, ganz besonders bevorzugt mindestens 10 mm, am meisten bevorzugt mindestens 100 mm.

Erfindungsgemäß bevorzugt beträgt die Änderung der Dehnungsstrecke höchstens 100 mm, besonders bevorzugt höchstens 10 mm, mehr bevorzugt höchstens 1000 µm, noch mehr bevorzugt mindestens 100 µm, sehr bevorzugt höchstens 10 µm, ganz besonders bevorzugt höchstens 1 µm, am meisten bevorzugt höchstens 0,5 µm.

Erfindungsgemäß bevorzugt beträgt die Geschwindigkeit der Änderung des Volumens mindestens so viel, dass an dem Gerüst anhaftende Zellen mindestens 1 µm/Tag distrahiert werden. Erfindungsgemäß bevorzugt beträgt die Geschwindigkeit der Änderung des Volumens höchstens so viel, dass an dem Gerüst anhaftende Zellen beziehungsweise osteogenes, kallusgebendes Gewebe höchstens 1 mm/Tag distrahiert werden. Eine schnellere Distraktionsfrequenz als 1 mm/Tag führt zur Differenzierung von Bindegewebe anstelle von Knochen. Durch die Volumenveränderung übermittelt das Gerüst an die im Blutkoagel enthaltenen und an dem Gerüstmaterial, auch im inneren des Gerüsts, adhärierten Zellen biomechanische Reize, die körpereigene Regenerationskräfte auslösen, wodurch sich neues autologes Knochenmaterial bildet. Dieses unterscheidet sich nicht von dem ursprünglichen Knochenmaterial, das den Defekt umgibt. Durch die Volumenveränderung des dreidimensionalen Gerüsts ergibt sich eine biomechanische Reizübertragung über den ganzen Raum, der von dem Gerüst eingenommen wird, hinweg, sodass auf wesentlich mehr Zellen ein biomechanischer Reiz übertragen wird als bei einer Distraktionsosteogenese aus dem Stand der Technik. Erfindungsgemäß bevorzugt wird der biomechanische Reiz von dem Gerüst direkt auf Osteoblasten übertragen.

Bei einer erfindungsgemäßen Distraktion können die biomechanischen Reize erfindungsgemäß bevorzugt nicht nur direkt an Osteoblasten, die an dem Gerüstmaterial anheften, sondern auch indirekt durch Fibroblasten übertragen werden. An das Gerüst anhaftende Fibroblasten übertragen erfindungsgemäß bevorzugt den Distraktionsreiz dosiert an Osteoblasten weiter. Ohne an die Theorie gebunden zu sein, werden nach Abschluss der Distraktion auch die Fibroblasten in der so genannten Nullzone zu Osteoblasten und bilden ebenfalls Knochen. Bei einer abnehmenden Distraktionsgeschwindigkeit ändert sich die Anzahl der den Osteoblasten vorgeschalteten Fibroblasten.

Eine Distraktionsosteogenese aus dem Stand der Technik überträgt dagegen biomechanische Reize über eine zweidimensionale Grenzfläche aus Knochen oder einem anderen Material nur an diejenigen Zellen, die diese zweidimensionale Grenzfläche direkt kontaktieren.

Die Erfindung stellt somit ein Verfahren bereit, bei dem ein dreidimensionales Gerüst in einen Knochendefekt eingebracht wird und das dreidimensionale Gerüst im Knochendefekt sein Volumen verändert. Durch die Volumenveränderung werden biomechanische Reize auf Zellen, insbesondere Osteoblasten, die sich im Volumen des dreidimensionalen Gerüsts befinden, übertragen, wodurch die Zellen zur Knochenbildung angeregt werden. Das dreidimensionale Gerüst überträgt somit biomechanische Reize zur Ausnutzung körpereigener Regenerationskräfte.

Das erfindungsgemäße Verfahren ist somit eine dreidimensionale Distraktion. Unter einer "dreidimensionalen Distraktion" wird im Zusammenhang mit der vorliegenden Erfindung eine distraktive Knochenregeneration verstanden, bei der biomechanische Reize nicht nur an der Grenzfläche zu einem Knochenfragment, also zweidimensional, übertragen werden, sondern eine Übertragung von Reizen über ein bestimmtes Volumen hinweg, also dreidimensional, stattfindet.

Das erfindungsgemäße Verfahren nutzt als Bioreaktor die körpereigenen Wundheilungsmechanismen. Die Knochenbildung erfolgt somit unter natürlichen Bedingungen, sodass die notwendigen Aspekte wie Wachstumsfaktoren, Hormone, Zellzusammensetzung implizit berücksichtigt sind. Damit überwindet das erfindungsgemäße Verfahren sowohl Probleme, die durch die hochkomplexe Steuerung bei einer Knochenregeneration entstehen können, als auch die Probleme einer langsamen und komplizierten Knochenregeneration durch Distraktionsverfahren aus dem Stand der Technik.

Erfindungsgemäß bevorzugt wird der Knochendefekt vor dem Einbringen des Gerüsts aufgefrischt. Erfindungsgemäß bevorzugt wird bei dem erfindungsgemäßen Verfahren vor dem Einbringen des Gerüsts in einen Knochendefekt dieser Defekt chirurgisch aufgefrischt, insbesondere eine Blutung erzeugt. Durch die chirurgische Auffrischung und die erzeugte Blutung bildet sich im Defekt ein Blutkoagel.

Nach der chirurgischen Auffrischung des Knochendefekts wird erfindungsgemäß bevorzugt ein Gerüst, insbesondere ein erfindungsgemäßes Gerüst, in den Knochendefekt eingebracht. Das Gerüstmaterial des eingebrachten Gerüsts wird von dem gebildeten Blutkoagel umschlossen, insbesondere vollständig umschlossen, das heißt, insbesondere die Zwischenräume, sehr bevorzugt Poren, des Gerüsts füllen sich, besonders bevorzugt ganz, mit dem Blutkoagel. Erfindungsgemäß bevorzugt verändert das Gerüst also nach einem definierten Zeitpunkt sein Volumen. Erfindungsgemäß bevorzugt verändert das Gerüst nach einem Tag sein Volumen. Erfindungsgemäß bevorzugt verändert das Gerüst nach einer Woche sein Volumen. Durch die Volumenveränderung ändern sich erfindungsgemäß bevorzugt auch die Form und/oder Größe der Zwischenräume, insbesondere Poren, des Gerüsts, sodass das Gerüst in alle Richtungen, also dreidimensional, gleichzeitig und/oder nacheinander Reize an die über den Raum verteilten an das Gerüst anhaftenden Zellen abgibt. Dadurch wird das Blutkoagel nicht schrumpfen, sondern vergrößert sich entsprechend der Volumenvergrößerung des Gerüsts. Die durch das Gerüst aktivierten Zellen können sich zu proliferierenden Osteoblasten umwandeln, die extrazelluläre Matrix produzieren, und es kann ein Kallus entstehen, der anschließend verknöchert. Wenn das Gerüst erfindungsgemäß bevorzugt bioabbaubar ist, wird dieses anschließend resorbiert und/oder metabolisiert. Somit kann sich der Knochendefekt mit Knochengewebe füllen, das erfindungsgemäß bevorzugt durch die beschriebenen biomechanischen Reize des Gerüsts entstanden ist. Dabei kann erfindungsgemäß bevorzugt auf künstlich eingebrachte Knochenersatzstoffe, Wachstumsfaktoren und andere Substanze neben dem Gerüst verzichtet werden. Das neu gebildete Knochenmaterial unterscheidet sich erfindungsgemäß bevorzugt weder histologisch noch in seinem biologischen beziehungsweise medizinischen Wertigkeit von den ihm umgebenden ursprünglichen Knochen.

Da das erfindungsgemäße Gerüst erfindungsgemäß bevorzugt bioabbaubar ist, kann der Raum, der durch den Abbau des Gerüsts entsteht, für die extrazelluläre Matrix genutzt werden. Die Degradation des Gerüsts kann erfindungsgemäß bevorzugt so eingestellt sein, dass bereits nach wenigen Wochen das Gerüst, nachdem es die biomechanischen Reize abgegeben hat, degradiert und der entstandene Raum von der extrazellulären Matrix eingenommen wird.

Erfindungsgemäß bevorzugt wird im Rahmen des erfindungsgemäßen Verfahrens ein Gerüst verwendet, dessen Gerüstmaterial zelladhäsive Eigenschaften hat. Besonders bevorzugt hat die Oberfläche des Gerüstmaterials zelladhäsive Eigenschaften. Die Oberfläche des Gerüstmaterials im Gerüst spielt für das Einwachsen von Zellen aus dem Blutkoagulat eine große Rolle. Eine erfindungsgemäß bevorzugte Adhäsion der Zellen an das Gerüstmaterial kann durch seine Oberflächenchemie und Oberflächenphysik sowie durch die O-berflächentopographie beeinflusst werden. Erfindungsgemäß bevorzugt ist die Oberfläche des Gerüstmaterials hydrophil. Für die einwachsenden Zellen ist die Wechselwirkung zwischen der negativ geladenen Zellmembran und den elektrischen Eigenschaften der Gerüstmaterialoberfläche erfindungsgemäß bevorzugt.

Erfindungsgemäß bevorzugt wird ein bioabbaubares Gerüst in den Defektbereich eines Knochens eingebracht. Erfindungsgemäß bevorzugt beginnt die Resorption des Gerüstmaterials nach 6 Wochen nach dem Einbringen des Gerüsts in einen Defektbereich eines Knochens.

In einer weiteren Ausgestaltung betrifft die Erfindung ein weiteres erfindungsgemäßes Verfahren zur Knochenregeneration, insbesondere ein weiteres dreidimensionales Distraktionsverfahren, wobei insbesondere ein vorgenanntes dreidimensionales Gerüst in einen Defektbereich eines Knochen eingebracht und dort bewegt wird. In diesem Defektbereich wird das Gerüst von einem Blutkoagel eingeschlossen, das heißt die Oberflächen des Gerüsts kontaktieren die im Blutkoagel enthaltenen autologen Zellen. Die vom Gerüstmaterial umschlossenen Zwischenräume werden, erfindungsgemäß bevorzugt zumindest teilweise, besonders bevorzugt größtenteils, insbesondere ganz durch das Blutkoagel ausgefüllt. Da das Gerüst dreidimensional aufgebaut ist, kann die Gerüstoberfläche im gesamten Raum, der durch das Gerüst ausgefüllt wird, mit dem im Blutkoagel enthaltenen autologen Zellen in Kontakt kommen. Nach dem Einbringen des Gerüsts in den Defektbereich eines Knochens wird das Gerüst in dem Knochendefekt bewegt. Die Bewegung findet dabei kontrolliert und gerichtet statt, das heißt in eine vordefinierte Richtung mit einer definierten Geschwindigkeit. Durch die Bewegung übermittelt das Gerüst an die im Blutkoagel enthaltenen und an dem Gerüstmaterial adhärierten Zellen biomechanische Reize, die körpereigene Regenerationskräfte auslösen, wodurch sich neues autologes Knochenmaterial bildet. Dieses unterscheidet sich nicht von dem ursprünglichen Knochenmaterial, das den Defekt umgibt. Durch die Bewegung des dreidimensionalen Gerüsts ergibt sich eine solche biomechanische Reizübertragung über den ganzen Raum, der von dem Gerüst eingenommen wird, hinweg, sodass auf wesentlich mehr Zellen ein biomechanischer Reiz übertragen wird als bei einer Distraktionsosteogenese aus dem Stand der Technik.

Die Erfindung stellt somit ein Verfahren bereit, bei dem ein dreidimensionales Gerüst in ein Knochendefekt eingebracht wird und das dreidimensionale Gerüst im Knochendefekt bewegt wird. Eine Volumenänderung des Gerüsts ist in dieser Ausgestaltung nicht notwendig, aber möglich. Durch die Bewegung werden biomechanische Reize auf Zellen, insbesondere Osteoblasten, die sich im Volumen des dreidimensionalen Gerüsts befinden übertragen, wodurch die Zellen zur Knochenbildung angeregt werden. Das dreidimensionale Gerüst überträgt somit biomechanische Reize zur Ausnutzung körpereigener Regenerationskräfte.

Erfindungsgemäß bevorzugt ist ein Verfahren zur Regeneration eines Knochens, wobei mindestens ein dreidimensionales Gerüst, umfassend ein Gerüstfasern ausbildendes Gerüstmaterial und von den Gerüstfasern umschlossene Zwischenräume, in einen Defektbereich eines Knochens eingebracht wird, wobei das Gerüstmaterial biokompatibel ist und eine zelladhäsive Eigenschaft hat, und wobei das Gerüst nach dem Einbringen in den Defektbereich in Abhängigkeit einer Krafteinwirkung vordefiniert und kontrolliert in dem Knochendefekt bewegt wird.

Das Gerüst wird erfindungsgemäß bevorzugt mit einer Geschwindigkeit von mindestens 1 µm/Tag und/oder höchstens 1,5 mm pro Tag, besonders bevorzugt von höchstens 1 mm pro Tag bewegt. Erfindungsgemäß bevorzugt erfolgt die Bewegung kontinuierlich oder diskontinuierlich.

Erfindungsgemäß bevorzugt wird ein bioabbaubares Gerüst in den Defektbereich eines Knochens eingebracht. Erfindungsgemäß bevorzugt beginnt die Resorption des Gerüstmaterials nach 6 Wochen nach dem Einbringen des Gerüsts in einen Defektbereich eines Knochens.

Erfindungsgemäß bevorzugt ist das verwendete dreidimensionale Gerüst ein erfindungsgemäßes dreidimensionales Gerüst. Erfindungsgemäß bevorzugt ändert sich das Volumen des dreidimensionalen Gerüsts bei dem Verfahren während des Bewegens des Gerüstes nicht.

Erfindungsgemäß bevorzugt kann das erfindungsgemäße Verfahren zur Reizübertragung mittels Volumenänderung eines dreidimensionalen Gerüsts mit dem erfindungsgemäßen Verfahren zur Reizübertragung mittels Bewegung eines dreidimensionalen Gerüsts kombiniert werden. Die offenbarten bevorzugten Merkmale des erfindungsgemäßen Verfahrens zur Reizübertragung mittels Volumenänderung sind insbesondere auch bevorzugte Merkmale des erfindungsgemäßen Verfahren zur Reizübertragung mittels Bewegung.

Erfindungsgemäß bevorzugt kann die Bewegung des dreidimensionalen Gerüsts durch mindestens eine von außen zugeführte Kraft ausgeführt werden. Erfindungsgemäß bevorzugt wird die Kraft mittels eines Zugseils oder Zugstabs zugefügt. Erfindungsgemäß bevorzugt ist die zugeführte Kraft Ultraschall. Erfindungsgemäß bevorzugt wird die Kraft durch einen Magneten zugeführt.

Die Erfindung betrifft auch die Verwendung eines biokompatiblen Gerüstmaterials, das vordefiniert und kontrolliert in Abhängigkeit von einer inneren oder äußeren Krafteinwirkung dehn- und/oder schrumpfbar ist und eine zelladhäsive Eigenschaft hat, zur Herstellung eines dreidimensionalen Gerüsts, umfassend Gerüstfasern aus dem Gerüstmaterial und von diesen umschlossene Zwischenräume, zur Regeneration eines Knochens, wobei das dreidimensionale Gerüst in einen Defektbereich eines Knochens eingebracht wird beziehungsweise werden kann.

Weitere vorteilhafte Ausgestaltungen der Erfindung ergeben sich aus den Unteransprüchen. Die Erfindung wird anhand des folgenden Ausführungsbeispiels und der dazugehörigen Figuren näher erläutert.
- Die Figur 1: zeigt einen Kit umfassend dreidimensionale Gerüste in einem Applikator in Form einer Spritze.
- Die Figur 2: zeigt schematisch ein in einen Knochendefekt eingebrachtes dreidimensionales Gerüst vor und nach Volumenänderung.

### Beispiel

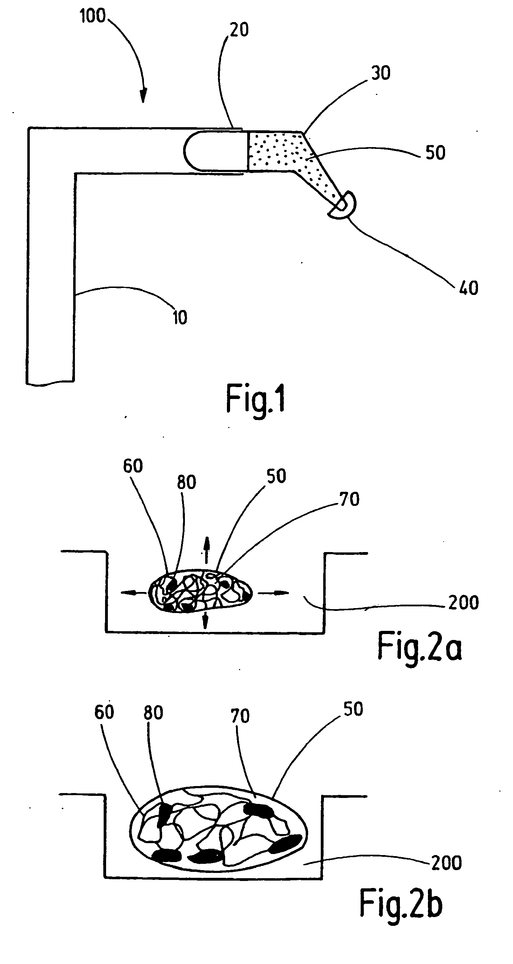

Die Figur 1 zeigt einen Kit 100, enthaltend eine Applikatorspritze 10 aus sterilisierbarem Metall, an deren offenem Ende 20 eine Einmalkapsel 30, zum Beispiel aus Kunststoff, aufgesteckt ist. Die Einmalkapsel 30 ist nach außen hin mit einer Schutzkappe 40 versehen. In der Einmalkapsel 30 befindet sich eine Mehrzahl von dreidimensionalen Gerüsten 50 in Form eines Granulats. Diese werden mittels der Spritze in einen nicht dargestellten Knochendefekt, zum Beispiel im Kieferbereich, eingespritzt.

Der erfindungsgemäße Kit 100 dient dazu, das Granulat aus den dreidimensionalen Gerüsten 50 in einen Knochendefekt einzuspritzen. Nach Einbringen in den Knochendefekt ändert sich aufgrund der erfindungsgemäßen Struktur und Zusammensetzung des Gerüstmaterials das Volumen des dreidimensionalen Gerüsts 50 und distrahiert die zwischenzeitlich in das Gerüst eingelagerten Knochenzellen zur Regeneration des Knochens.

Figur 2a zeigt ein resorbierbares Gerüst 50 aus einem elastischen Polymer in einem Knochendefekt 200, und zwar unmittelbar nach Einbringen dieses Gerüsts 50 in den Knochendefekt 200, zum Beispiel mittels eines Kits 100. Das Gerüst 50 ist aus Gerüstfasern 60 und Zwischenräumen 70 aufgebaut. Eingelagert in die Zwischenräume 70 und anhaftend an Gerüstfasern 60 sind bereits einige osteogene Zellen, zum Beispiel Osteoblasten 80. Das Gerüst 50 aus dem elastischen Polymer wurde nach seiner Herstellung und vor dem Einbringen in den Defekt 200 zunächst mechanisch komprimiert und dann durch Eintauchen in ein Bad einer bioresorbierbaren Vernetzungslösung, zum Beispiel eines weiteren Polymers, in dieser komprimierten Form fixiert. Nach Einbringen in den Defekt 200 wird die bioresorbierbare Vernetzung konstant und vordefiniert resorbiert, wodurch sich, wie in Figur 2a durch die Pfeile schematisch dargestellt, das Gerüst 50 in alle drei Dimensionen räumlich dehnt, das heißt vergrößert, so dass sich, wie aus Figur 2b erkenntlich, ein volumenvergrößertes Gerüst 50 ergibt, sobald die bioresorbierbare Vernetzung gänzlich resorbiert wurde. Die Volumenvergrößerung führt zu einer Distraktion der in dem Gerüst 50 eingelagerten und adhärierten Zellen 80.

## Patentansprüche

1. Dreidimensionales Gerüst zur Regeneration eines Knochens, umfassend Gerüstfasern aus mindestens einem Gerüstmaterial mit plastischen oder elastischen Eigenschaften und von diesen umschlossene Zwischenräume, wobei die Gerüstfasern eine Dicke von 50 µm bis 3000 µm aufweisen und wobei mindestens ein Teil der Zwischenräume einen Durchmesser von 5 µm bis 25 µm aufweist, wobei das Gerüstmaterial biokompatibel ist, vordefiniert und kontrolliert in Abhängigkeit von einer Krafteinwirkung dehn- oder schrumpfbar ist und eine zelladhäsive Eigenschaft hat, und wobei das Gerüst über eine elastische Rückstellkraft verfügt, wodurch das Gerüst in Abhängigkeit der Krafteinwirkung vordefiniert und kontrolliert sein Ausgangsvolumen durch Füllen der Zwischenräume des Gerüsts mit einer Flüssigkeit vergrößern kann oder wobei das Gerüst ein bioabbaubares Formelement aufweist, aufgrund dessen sich das Gerüst in einem gedehnten oder komprimierten Ausgangsvolumen befindet, und wobei sich das Ausgangsvolumen durch Entfernung des Formelements ändern kann.

2. Gerüst nach Anspruch 1, wobei sich das Ausgangsvolumen durch Füllen der Zwischenräume des Gerüsts mit einer Flüssigkeit enthaltend Biomoleküle und/oder Zellen, besonders bevorzugt Blut, vergrößern kann.

3. Gerüst nach Anspruch 1 oder 2, wobei das Formelement eine Ummantelung, eine Umschnürung oder einen Kleber ist.

4. Gerüst nach einem der vorhergehenden Ansprüche, wobei das Material des Formelements, nämlich der Ummantelung, des Klebers und/oder der Umschnürung ausgewählt ist aus der Gruppe bestehend aus Fibrin, Kollagen, mindestens einem Polysaccharid und Mischungen davon.

5. Gerüst nach einem der vorhergehenden Ansprüche, wobei das Gerüstmaterial ein Material enthält, ausgewählt aus der Gruppe bestehend aus Polyglykolsäure, Polymilchsäure, Poly(ε-caprolacton), Poly(β-hydroxybutyrat), Poly(p-dioxanon), einem Polyanhydrid oder einer Mischung von diesen.

6. Gerüst nach einem der vorhergehenden Ansprüche, wobei das Gerüstmaterial aus einem Material besteht, ausgewählt aus der Gruppe bestehend aus Polyglykolsäure, Polymilchsäure, Poly(ε-caprolacton), Poly(β-hydroxybutyrat), Poly(p-dioxanon), einem Polyanhydrid oder einer Mischung von diesen.

7. Gerüst nach einem der vorhergehenden Ansprüche, wobei das Gerüstmaterial aus Polymilchsäure und Polyglykolsäure besteht.

8. Gerüst nach einem der vorhergehenden Ansprüche, wobei das Gerüstmaterial anisotrop ist.

9. Gerüst nach einem der vorhergehenden Ansprüche, wobei das Gerüstmaterial Fasern eines Faserverbundwerkstoffs enthält.

10. Gerüst nach einem der vorhergehenden Ansprüche, wobei die Gerüstfasern des Gerüstmaterials eine Dicke von 60 µm bis 2000 µm aufweisen.

11. Gerüst nach einem der vorhergehenden Ansprüche, wobei die Zwischenräume als Poren ausgebildet sind.

12. Gerüst nach einem der vorhergehenden Ansprüche, wobei die Zwischenräume einen Durchmesser von 5 µm bis 25 µm haben.

13. Gerüst nach einem der Ansprüche 1 bis 11, wobei ein Teil der Zwischenräume einen Durchmesser von 0,5 µm bis 5 µm, ein Teil der Zwischenräume einen Durchmesser von 5 µm bis 25 µm und ein Teil der Zwischenräume einen Durchmesser von 100 µm bis 1000 µm hat.

14. Gerüst nach einem der vorhergehenden Ansprüche, wobei das Gerüstmaterial bioabbaubar ist.

15. Granulat, bestehend aus mindestens 2 Gerüsten nach einem der Ansprüche 1 bis 14.

16. Gerüst nach einem der Ansprüche 1 bis 14 oder Granulat nach Anspruch 15 zur Verwendung zur Regeneration eines Knochens.

17. Verwendung eines dreidimensionalen Gerüsts nach einem der Ansprüche 1 bis 14 oder eines Granulats nach Anspruch 15 zur Herstellung eines Kits zur Knochenregeneration.

18. Verfahren zur Herstellung eines dreidimensionalen Gerüsts nach einem der Ansprüche 1 bis 14, wobei Gerüstfasern aus mindestens einem nicht-biogenen Gerüstmaterial in einem Fließpressverfahren zu einem Gerüst geformt werden, wobei das Gerüstmaterial biokompatibel ist, vordefiniert und kontrolliert in Abhängigkeit von einer Krafteinwirkung dehn- und/oder schrumpfbar ist und eine zelladhäsive Eigenschaft hat.

## Claims

1. Three-dimensional framework for the regeneration of a bone, comprising framework fibres composed of at least one framework material having plastic or elastic properties, and interspaces enclosed by same, wherein the framework fibres have a thickness of 50 µm to 3000 µm and wherein at least one portion of the interspaces has a diameter of 5 µm to 25 µm, wherein the framework material is biocompatible, and expandable or shrinkable in a predefined and controlled manner as a function of a force effect and has a cell adhesive property, and wherein the framework has an elastic restoring force, whereby the framework may enlarge its starting volume in a predefined and controlled manner as a function of the force effect by filling the interspaces of the framework with a liquid, and wherein the framework has a biodegradable moulded element by means of which the framework is in an expanded or compressed starting volume, and wherein the starting volume may be changed by removing the moulded element.

2. Framework according to claim 1, wherein the starting volume may be enlarged by filling the interspaces of the framework with a liquid containing biomolecules and/or cells, particularly preferably blood.

3. Framework according to claim 1 or 2, wherein the moulded element is a casing, a confinement or an adhesive.

4. Framework according to one of the preceding claims, wherein the material of the moulded element, namely of the casing, of the adhesive and/or of the confinement, is selected from the group consisting of fibrin, collagen, at least one polysaccharide, and mixtures thereof.

5. Framework according to one of the preceding claims, wherein the framework material contains a material selected from the group consisting of polyglycolic acid, polylactic acid, poly(ε-caprolactone), poly(β-hydroxybutyrate), poly(p-dioxanone), a polyanhydride, or a mixture thereof.

6. Framework according to one of the preceding claims, wherein the framework material consists of a material selected from the group consisting of polyglycolic acid, polylactic acid, poly(ε-caprolactone), poly(β-hydroxybutyrate), poly(p-dioxanone), a polyanhydride, or a mixture thereof.

7. Framework according to one of the preceding claims, wherein the framework material consists of polylactic acid and polyglycolic acid.

8. Framework according to one of the preceding claims, wherein the framework material is anisotropic.

9. Framework according to one of the preceding claims, wherein the framework material contains fibres of a fibre composite.

10. Framework according to one of the preceding claims, wherein the framework fibres of the framework material have a thickness of 60 µm to 2000 µm.

11. Framework according to one of the preceding claims, wherein the interspaces are designed as pores.

12. Framework according to one of the preceding claims, wherein the interspaces have a diameter of 5 µm to 25 µm.

13. Framework according to one of claims 1 through 11, wherein one portion of the interspaces has a diameter of 0.5 µm to 5 µm, one portion of the interspaces has a diameter of 5 µm to 25 µm, and one portion of the interspaces has a diameter of 100 µm to 1000 µm.

14. Framework according to one of the preceding claims, wherein the framework material is biodegradable.

15. Granulate consisting of at least two frameworks according to one of claims 1 through 14.

16. Framework according to one of claims 1 through 14 or granulate according to claim 15 for use for the regeneration of a bone.

17. Use of a three-dimensional framework according to one of claims 1 through 14 or a granulate according to claim 15 for producing a bone regeneration kit.

18. Method for producing a three-dimensional framework according to one of claims 1 through 14, wherein framework fibres composed of at least one non-biogenic framework material are formed into a framework in an extrusion process, wherein the framework material is biocompatible and is expandable and/or shrinkable in a predefined and controlled manner as a function of a force effect, and has a cell adhesive property.

## Revendications

1. Structure tridimensionnelle destinée à la régénération d'un os, comprenant des fibres de structure composées d'au moins un matériau de structure ayant des propriétés plastiques ou élastiques et d'interstices entourés de celles-ci, les fibres de structure présentant une épaisseur de 50 µm à 3000 µm et au moins une partie des interstices présentant un diamètre de 5 µm à 25 µm, le matériau de structure étant biocompatible, extensible ou rétractable de façon prédéfinie et contrôlée en fonction de l'application d'une force et ayant une propriété d'adhésivité aux cellules, et la structure ayant une force de rappel élastique, ce qui permet que la structure augmente son volume de sortie par remplissage des interstices de la structure avec un liquide de façon prédéfinie et contrôlée en fonction de l'application d'une force ou la structure présentant un élément de moulage biodégradable, lequel force la structure dans un volume de sortie étendu ou comprimé, et le volume de sortie pouvant être modifié en détachant l'élément de moulage.

2. Structure selon la revendication 1, le volume de sortie pouvant augmenter en remplissant les interstices de la structure avec un liquide contenant des biomolécules et/ou des cellules, de préférence du sang.

3. Structure selon la revendication 1 ou 2, l'élément de moulage étant une gaine, un frettage ou un adhésif.

4. Structure selon l'une quelconque des revendications précédentes, le matériau de l'élément de moulage, à savoir la gaine, l'adhésif et/ou le frettage, étant sélectionné dans le groupe consistant en fibrine, collagène, au moins un polysaccharide et des mélanges de ceux-ci.

5. Structure selon l'une quelconque des revendications précédentes, le matériau de structure contenant un matériau sélectionné dans la groupe consistant en acide polyglycolique, acide polylactique, poly(ε-caprolactone), poly(β-hydroxybutyrate), poly(p-dioxanone), un polyanhydride ou un mélange de ceux-ci.

6. Structure selon l'une quelconque des revendications précédentes, le matériau de structure étant composé d'un matériau sélectionné dans la groupe consistant en acide polyglycolique, acide polylactique, poly(ε-caprolactone), poly(β-hydroxybutyrate), poly(p-dioxanone), un polyanhydride ou un mélange de ceux-ci.

7. Structure selon l'une quelconque des revendications précédentes, le matériau de structure étant composé d'acide polylactique et d'acide polyglycolique.

8. Structure selon l'une quelconque des revendications précédentes, le matériau de structure étant anisotrope.

9. Structure selon l'une quelconque des revendications précédentes, le matériau de structure contenant des fibres d'un matériau composite fibreux.

10. Structure selon l'une quelconque des revendications précédentes, les fibres de structure du matériau de structure présentant une épaisseur de 60 µm à 2000 µm.

11. Structure selon l'une quelconque des revendications précédentes, les interstices étant configurés sous forme de pores.

12. Structure selon l'une quelconque des revendications précédentes, les interstices ayant un diamètre de 5 µm à 25 µm.

13. Structure selon l'une quelconque des revendications 1 à 11, une partie des interstices ayant un diamètre de 0,5 µm à 5 µm, une partie des interstices ayant un diamètre de 5 µm à 25 µm et une partie des interstices ayant un diamètre de 100 µm à 1000 µm.

14. Structure selon l'une quelconque des revendications précédentes, le matériau de structure étant biodégradable.

15. Granulat composé d'au moins deux structures selon l'une quelconque des revendications 1 à 14.

16. Structure selon l'une quelconque des revendications 1 à 14 ou granulat selon la revendication 15 destiné(e) à être utilisé(e) pour la régénération d'un os.

17. Utilisation d'une structure tridimensionnelle selon l'une quelconque des revendications 1 à 14 ou d'un granulat selon la revendication 15 pour la fabrication d'un kit de régénération osseuse.

18. Procédé de fabrication d'une structure tridimensionnelle selon l'une quelconque des revendications 1 à 14, des fibres de structure composées d'au moins un matériau de structure non biogène étant moulées au cours d'un procédé de presse à extrusion pour former une structure, le matériau de structure étant biocompatible, extensible et/ou rétractable de façon prédéfinie et contrôlée en fonction de l'application d'une force et ayant une propriété d'adhésivité aux cellules.
